# DEMANDE DE BREVET EUROPEEN

(11) **EP 1 872 769 A1**
(43) Date de publication de la demande: **02.01.2008**
(21) Numéro de dépôt: 07109706.7
(22) Date de dépôt: 06.06.2007
(51) Int. Cl.: A61K 8/11, A61K 8/46, A61K 8/49, A61Q 5/10

(54) **Utilisation de 3h-1,2-dithiole-3-thione, d'anethole dithiolethione, de sulforaphane, de phenethyl isothiocyanate, de 6-methyl-sulphinyl)hexyl isothiocyanate et d'allyl isothiocyanate pour le traitement de la canitie**

(30) Priorité: 20.06.2006 FR 0652555
(71) Demandeur: L'ORÉAL, 75008 Paris (FR)
(72) Inventeur: COMMO, Stéphane, 92310, SEVRES (FR)
(74) Mandataire: Bernstein, Claire Jacqueline

(57) **Abrégé**

La présente invention se rapporte à l'utilisation de 3H-1,2-dithiole-3-thione, d'anethole dithiolethione, de sulforaphane, de phenetyl isothiocyanate, de 6-(methylsulphinyl)hexyl isothiocyanate et d'allyl isothiocyanate et leurs dérivés pour le traitement de la canitie et à des compositions comprenant au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés et leur mélange associé à un autre actif choisi parmi les agents de lutte des états desquamatifs du cuir chevelu, des extraits végétaux à activité propigmentante et un agent ralentissant la chute des cheveux ou favorisant leur repousse.

## Description

La présente invention se rapporte à l'utilisation cosmétique de 3H-1,2-dithiole-3-thione (D3T), d'anethole dithiolethione (ADT), de sulforaphane, de phenetyl isothiocyanate, de 6-(methylsulphinyl)hexyl isothiocyanate et d'allyl isothiocyanate et de leurs dérivés et leur mélange pour traiter la canitie.

Le follicule pileux est une invagination tubulaire de l'épiderme qui s'enfonce jusqu'aux couches profondes du derme. La partie inférieure, ou bulbe pileux, comporte elle-même une invagination dans laquelle se trouve la papille dermique. La partie inférieure du bulbe est une zone de prolifération cellulaire où se trouvent les précurseurs des cellules kératinisées constituant le cheveu. Les cellules en ascension issues de ces précurseurs se kératinisent progressivement dans la partie supérieure du bulbe, et cet ensemble de cellules kératinisées formera la tige pilaire.

La couleur des cheveux et des poils repose notamment sur la présence en quantités et ratios variables de deux groupes de mélanines : les eumélanines (pigments bruns et noirs) et les phéomélanines (pigments rouges et jaunes). La pigmentation du cheveu et des poils requiert la présence de mélanocytes au niveau du bulbe du follicule pileux. Ces mélanocytes sont dans un état actif, c'est-à-dire qu'ils synthétisent des mélanines. Ces pigments sont transmis aux kératinocytes destinés à former la tige pilaire ce qui conduira à la pousse d'un cheveu ou d'un poil pigmenté. Cette structure est appelée ci-après « unité folliculaire de pigmentation ».
Chez les mammifères, la mélanogénèse implique au moins trois enzymes : la tyrosinase, la DOPAchrome tautomérase (TRP-2, pour Tyrosinase Related Protein 2) et la DHICAoxydase (TRP-1, pour Tyrosinase Related Protein 1).
La tyrosinase est l'enzyme qui initie la biosynthèse des mélanines. Elle est également décrite comme étant l'enzyme limitante de la mélanogénèse.
La TRP-2 catalyse la tautomérisation du DOPAchrome en acide 5,6-Dihydroxyindole-2-carboxylique (DHICA). En l'absence de TRP-2, le DOPAchrome subit une décarboxylation spontanée pour former le 5,6-dihydroxyindole (DHI).
DHICA et DHI sont tous deux des précurseurs de pigments, TRP-1 oxyde les molécules de DHICA pour former des dérivés de quinones (Pawelek JM and Chakraborty AK. The enzymology of melanogenesis. In: Nordlund JJ, Boissy RE, Hearing VJ, King RA, Ortonne J-P. The Pigmentary System: Physiology and Pathophysiology. New York: Oxford university press; 1998. p. 391-400).

Les trois enzymes, tyrosinase, TRP-2 et TRP-1, apparaissent spécifiquement impliquées dans la mélanogénèse. De plus, l'activité de ces trois enzymes a été décrite comme nécessaire à l'activité maximale de biosynthèse des eumélanines.

Le cheveu et le poil subissent un cycle. Ce cycle comprend une phase de croissance (phase anagène), une phase de dégénérescence (phase catagène) et une phase de repos (phase télogène) à la suite de laquelle une nouvelle phase anagène se développera. En raison de ce cycle pilaire, et contrairement à l'unité de pigmentation épidermique, l'unité folliculaire de pigmentation doit également être cycliquement renouvelée.

La canitie (blanchissement naturel des cheveux) est liée à une raréfaction spécifique et progressive des mélanocytes des cheveux affectant à la fois les mélanocytes du bulbe pileux et les cellules précurseur de mélanocytes (Commo et al. Br J Dermatol 2004 ;150 :435-443). D'autres types cellulaires présents dans les follicules pileux ne sont pas affectés. De plus, cette raréfaction de mélanocytes n'est pas observée dans l'épiderme. La cause de cette raréfaction progressive et spécifique de mélanocytes et précurseurs de mélanocytes dans le follicule pileux n'est à ce jour pas identifiée.

Il apparaît donc nécessaire de lutter contre la disparition des mélanocytes des follicules pileux humains, processus affectant à la fois les mélanocytes actifs des bulbes et les mélanocytes quiescents de la région supérieure des follicules pileux, pour lutter contre la canitie.

La Demanderesse a identifié un moyen de lutter contre le blanchissement des cheveux en agissant sur l'enzyme TRP-2 (WO 03/103568) notamment par l'augmentation du taux de GSH. En effet, elle a mis en évidence que l'expression de l'enzyme TRP-2 est corrélée à un taux plus élevé de GSH dans les mélanocytes, l'expression de TRP-2 induit une augmentation du taux de GSH dans les mélanocytes. Ainsi dans les mélanocytes qui n'expriment pas TRP-2 (par exemple, les précurseurs de mélanocytes du cheveu), il y a un taux de GSH bas en comparaison des mélanocytes qui expriment l'enzyme TRP-2 (par exemple, tous les mélanocytes de la peau).
La Demanderesse a donc identifié une nouvelle cible pour le traitement de la canitie, plus particulièrement, elle a mis en évidence que les composés capables d'augmenter le taux de GSH dans les mélanocytes déficients en TRP-2 augmentent la viabilité de ces mélanocytes, diminuent le blanchissement des cheveux et conduisent, contrairement à leur effet dépigmentant décrit dans la littérature, à la restauration de la pigmentation des cheveux (FR04/13756).

La Demanderesse a maintenant mis en évidence que le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés, par leur capacité d'augmenter le taux de GSH dans les mélanocytes, s'opposent au blanchissement des cheveux et conduisent à la restauration de la pigmentation des cheveux.

L'objet de la présente invention se rapporte à l'utilisation d'au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et de leurs dérivés comme agent permettant de prévenir, limiter ou arrêter la progression de la canitie, et maintenir et/ou favoriser la re-pigmentation naturelle des cheveux et/ou des poils.

Le 3H-1,2-dithiole-3-thione (D3T) et l'anethole dithiolethione (ADT) sont des composés de la famille des dithiolethiones décrits commme augmentant le taux de GSH (dans Cuao Z et al. BBRC 317 (2004) 1080-1088 et Warnet et al. JM Pharmacol Toxicol 1989 65(1):63-4, respectivement).

Les composés dérivés d'isothiocyanate (ITC) :
- le sulforaphane 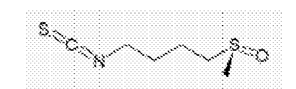 et son dérivé glucosinolate, encore appelé glucoraphanin, naturellement présent dans le brocoli, 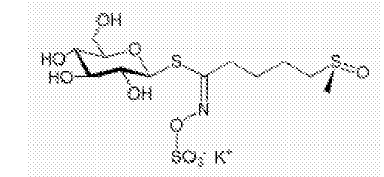
- le phenetyl isothiocyanate : 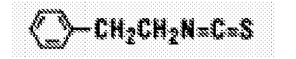 et son glucosinolate (le gluconasturiin) : 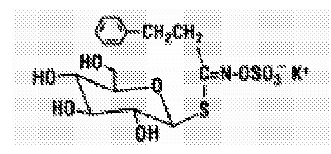
- le 6-(methylsulphinyl)hexyl isothiocyanate est un dérivé du sulforaphane présent dans la racine de Wasabi ;
- l'allyl isothiocyanate (3-isothiocyanato-1-propene) ; 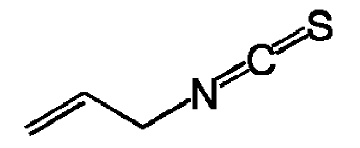 sont décrits dans la littérature pour augmenter le taux de GSH (Ye et al. Carcinogenesis, 2001 vol.22, no.12 pp.1987-1992).
   Dans une variante de l'invnetion, on préférera utiliser les dérivés des ICT naturellement présents dans les végétaux (crucifère), il s'agit généralement des dérivés glucosinolates.

En particulier, l'objet de l'invention concerne l'utilisation d'au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés pour prévenir et/ou limiter et/ou arrêter le développement de la canitie.
Ces composés pourront également être utilisés en mélange.

L'objet de l'invention se rapporte aussi à l'utilisation d'au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés pour maintenir la pigmentation naturelle des cheveux et/ou des poils gris.

Un objet de l'invention est également une composition pour lutter contre la canitie, comprenant dans un milieu cosmétiquement acceptable, au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés associé à un autre actif capillaire choisi parmi les agents de lutte des états desquamatifs du cuir chevelu et/ou des extraits végétaux à activité pro-pigmentante.

La présente invention se rapporte aussi à une composition pour lutter contre la canitie comprenant dans un milieu cosmétiquement acceptable, au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés, associé à un agent ralentissant la chute des cheveux ou favorisant leur repousse.

La composition selon l'invention comprend une quantité d'au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés comprise entre 0,001 et 10% en poids par rapport au poids total de la composition, préférentiellement entre 0,01 et 5% en poids par rapport au poids total de la composition et encore plus préférentiellement entre 0,1 et 1% en poids par rapport au poids total de la composition.

La composition selon l'invention peut être administrée par voie orale ou appliquée topiquement sur la peau (sur toute zone cutanée du corps recouverte de poils) et/ou le cuir chevelu.

Par voie orale, la composition selon l'invention peut contenir le ou les composés choisis parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés en solution dans un liquide alimentaire tel qu'une solution aqueuse ou hydroalcoolique, éventuellement aromatisée. Ils peuvent également être incorporés dans un excipient solide ingérable et se présenter par exemple sous forme de granulés, de pilules, de comprimés ou de dragées. Ils peuvent également être placés en solution dans un liquide alimentaire conditionné lui-même éventuellement dans des capsules ingérables.

Selon le mode d'administration, la composition de l'invention peut se présenter sous toutes les formes galéniques normalement utilisées, particulièrement en cosmétologie.
Une composition préférée de l'invention est une composition cosmétique adaptée à une application topique sur le cuir chevelu et/ou la peau.

Pour une application topique, la composition utilisable selon l'invention peut être notamment sous la forme d'une solution aqueuse, hydroalcoolique ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Elle peut ainsi se présenter sous forme d'onguent, de teinture, de crème, de pommade, de poudre, de timbre, de tampon imbibé, de solution, d'émulsion ou de dispersion vésiculaire, de lotion, de gel, de spray, de suspension, de shampooing, d'aérosol ou de mousse. Elles peuvent être anhydres ou aqueuses. Elle peut également consister en des préparations solides constituant des savons ou des pains de nettoyage.
Ces compositions sont préparées selon les méthodes usuelles.

La composition utilisable selon l'invention peut en particulier être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, de masque.

La composition cosmétique selon l'invention sera préférentiellement une crème, une lotion capillaire, un shampooing ou un après-shampooing.

Les quantités des différents constituants des compositions utilisables selon l'invention sont celles classiquement utilisées dans les domaines considérés.

Lorsque la composition utilisable selon l'invention est une émulsion, la proportion de la phase grasse peut aller de 5% à 80% en poids, et de préférence de 5% à 50% en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition utilisable selon l'invention est une solution ou un gel huileux, la phase grasse peut représenter plus de 90% du poids total de la composition.

Dans une variante de l'invention, la composition sera telle que le composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés est encapsulé dans un enrobage tel que des microsphères, des nanosphères, des oléosomes ou des nanocapsules.
Ce type de formulation s'avère avantageux car il permet de cibler spécifiquement le follicule pileux et ainsi la libération de l'actif sur son site d'action.

A titre d'exemple, les microsphères pourront être préparées selon la méthode décrite dans la demande de brevet EP 0 375 520.

Les nanosphères pourront se présenter sous forme de suspension aqueuse et être préparées selon les méthodes décrites dans les demandes de brevet FR 0015686 et FR 0101438.

Les oléosomes consistent en une émulsion huile dans eau formée par des globules huileux pourvus d'un enrobage cristal liquide lamellaire dispersé dans une phase aqueuse (voir les demandes de brevet EP 0 641 557 et EP 0 705 593).

Le composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés pourra aussi être encapsulé dans des nanocapsules consistant en un enrobage lamellaire obtenu à partir d'un tensio-actif siliconé (voir la demande de brevet EP 0 780 115), les nanocapsules pourront également être préparées à base de polyesters sulfoniques hydrodispersibles (voir la demande de brevet FR 0113337).

Le composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés pourra également être complexé à la surface de globules huileux cationiques, quelques soit leur taille (voir les demandes de brevet EP 1 010 413, EP 1 010 414, EP 1 010 415, EP 1 010 416, EP 1 013 338, EP 1 016 453, EP 1 018 363, EP 1 020 219, EP 1 025 898, EP 1 120 101, EP 1 120 102, EP 1 129 684, EP 1 160 005 et EP 1 172 077).

Le composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés peut enfin être complexé à la surface de nanocapsules ou nanoparticules pourvues d'un enrobage lamellaire (Voir EP 0 447 318 et EP 0 557 489) et contenant un tensio-actif cationique à la surface (voir les références citées précédemment pour les tensio-actifs cationiques).

En particulier, on préférera une composition telle que l'enrobage contenant le composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés a un diamètre inférieur ou égale à 10 µm. Lorsque l'enrobage ne forme pas une vésicule sphérique, on entend par diamètre la dimension la plus grande de la vésicule.

De façon connue, la composition selon l'invention peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01% à 10% du poids total de la composition. Ces adjuvants, selon leur nature, peuvent être introduits dans la phase grasse, dans la phase aqueuse et/ou dans les sphérules lipidiques.

Les compositions utilisables selon l'invention peuvent associer au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés à d'autres agents actifs. Parmi ces agents actifs, on peut citer à titre d'exemple :
- les agents modulant la différenciation et/ou la prolifération et/ou la pigmentation des cellules de la peau tels que le rétinol et ses esters, la vitamine D et ses dérivés, les oestrogènes tels que l'oestradiol, les modulateurs de l'AMPc tels que les dérivés de POMC, l'adénosine, ou la forskoline et ses dérivés, les prostaglandines et leurs dérivés, la triiodotrionine et ses dérivés ;
- des extraits de végétaux tels que ceux d'Iridacées ou de soja, extraits pouvant alors contenir ou non des isoflavones ;
- des extraits de micro-organismes ;
- les agents anti-radicaux libres tels que l'α-tocophérol ou ses esters, les superoxyde dismutases ou ses mimétiques, certains chélatants de métaux ou l'acide ascorbique et ses esters ;
- les anti-séborrhéiques tels que certains acides aminés soufrés, l'acide 13-cis rétinoïque, l'acétate de cyprotérone ;
- les autres agents de lutte contre les états desquamatifs du cuir chevelu comme le zinc pyrithione, le disulfure de sélénium, le climbazole, l'acide undécylénique, le Kétoconazole, la piroctone olamine (octopirox) ou la ciclopiroctone (ciclopirox) ;
   en particulier, il pourra s'agir d'actifs stimulant la repousse et/ou favorisant le ralentissement de la chute des cheveux, on peut plus particulièrement citer à titre non limitatif :
- les esters d'acide nicotinique, dont notamment le nicotinate de tocophérol, le nicotinate de benzyle et les nicotinates d'alkyles en C₁-C₆ comme les nicotinates de méthyle ou d'hexyle ;
- les dérivés de pyrimidine, comme le 2,4-diamino 6-piperidinopyrimidine 3-oxyde ou "Minoxidil" décrit dans les brevets US 4,139,619 et US 4,596,812 ; l'Aminexil ou 2,4 diamino pyrimidine 3 oxyde décrit dans WO96/09048 ;
- les agents inhibiteur de la lipoxygenase ou inducteur de la cyclooxydase favorisant la repousse des cheveux comme ceux décrits par la Demanderesse dans la demande de brevet européen EP 0 648 488 ;
- les agents antibactériens tels que les macrolides, les pyranosides et les tétracyclines, et notamment l'Erythromycine ;
- les agents antagonistes de calcium, comme la Cinnarizine, la Nimodipine et la Nifedipine ;
- des hormones, telles que l'estriol ou des analogues, ou la thyroxine et ses sels ;
- des agents antiandrogènes, tels que l'oxendolone, la spironolactone, le diéthylstilbestrol et la flutamide ;
- des inhibiteurs stéroïdiens ou non stéroïdiens des 5-α-réductases tels que ceux décrits par la Demanderesse dans les demandes de brevet européen EP 0 964 852 et EP 1 068 858 ou encore le finastéride ;
- des agonistes des canaux potassiques dépendant de l'ATP tels que la cromakalim et le nicorandil ;
- des extraits végétaux à activité pro-pigmentante comme les extraits de chrysanthème tels que décrits dans FR 2768343 et les extraits de Sanguisorba décrits dans FR 2782920.

De préférence, le composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés est associé à au moins un autre actif capillaire choisi parmi les agents de lutte des états desquamatifs du cuir chevelu, des agents ralentissant la chute des cheveux ou favorisant leur repousse, des extraits végétaux à activité propigmentante.

Un autre objet de la présente invention se rapporte à un procédé de traitement cosmétique de la canitie caractérisé en ce qu'on administre ou qu'on applique sur la zone à traiter une composition telle que définie précédemment comprenant au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés.

La présente invention se rapporte encore à un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés pour son utilisation dans le traitement de la canitie.

L'invention se rapporte aussi à un procédé de traitement cosmétique destiné à maintenir la pigmentation naturelle des cheveux et/ou des poils gris ou blancs caractérisé en ce qu'on administre ou qu'on applique sur la zone à traiter une composition telle que définie précédemment comprenant au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés.

Les procédés de traitement de la canitie et de pigmentation des cheveux et/ou des poils gris ou blancs peuvent également consister en l'ingestion d'une composition comprenant au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés.

Les zones à traiter peuvent être, par exemple et sans aucune limitation, le cuir chevelu, les sourcils, la moustache et/ou la barbe et toute zone de la peau recouverte de poils.

Plus particulièrement, les procédés de traitement cosmétique de la canitie et de pigmentation naturelle des cheveux et/ou poils gris ou blancs consistent à appliquer une composition comprenant au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés.

Les procédés de traitement cosmétique pour lutter contre la canitie et/ou pour maintenir la pigmentation naturelle des cheveux et/ou des poils gris ou blancs peut par exemple consister à appliquer la composition sur les cheveux et le cuir chevelu, le soir, garder la composition toute la nuit et éventuellement effectuer un shampooing le matin ou laver les cheveux à l'aide de cette composition et à laisser à nouveau en contact quelques minutes avant de rincer. La composition conforme à l'invention s'est révélée particulièrement intéressante lorsqu'elle est appliquée sous forme de lotion capillaire, éventuellement rincée ou même sous forme d'un shampooing.

### Exemple 1 - mesure de l'activité des composés utiles selon l'invention

### 1-A-1. Protocole de mesure des espèces réactives de l'oxygène (ROS) générés par un stress H₂O₂ dans des mélanocytes humains normaux (MHN) en culture

Les mélanocytes humains normaux (MHN) sont ensemencés à J0 à la densité de 4.10⁴ cellules/cm². A J1, le milieu de culture est remplacé par la solution de *6-carboxy-2',7'-dichlorodihydrofluoresceine diacetate, di[acetoxymethyl ester]* à 10 µM en PBS (H₂DCFDA, C2938, Molecular Probes). Après 20 min, la solution d'H₂DCFDA est remplacée par le milieu de culture. Les MHN sont laissés 30 min avant d'ajouter la solution d'H₂O₂ (250 µM). La fluorescence est mesurée après 15 min au fluoroscan (excitation : 485nm, émission : 538nm).

Les composés étudiés sont utilisés en fonctions de leurs propriétés (concentration, durée de pré incubation). La N-Acétyl-Cystéine (A9165, Sigma) est utilisée à titre de molécule de référence. Les mélanocytes sont prétraités 12h/18h à 37°C avec le composé étudié dans le milieu de culture, avant d'être mis en contact 20 minutes avec le H₂DCFDA (10µM en PBS). La solution d'H₂DCFDA est ensuite remplacée par du milieu de culture contenant l'actif étudié. Après 30 min d'incubation le stress oxydatif est induit par ajout de 250µM d'H₂O₂ dans le milieu de culture. La fluorescence est mesurée après 15 min au fluoroscan (excitation : 485nm, émission : 538nm).

### 1-A-2. Résultats

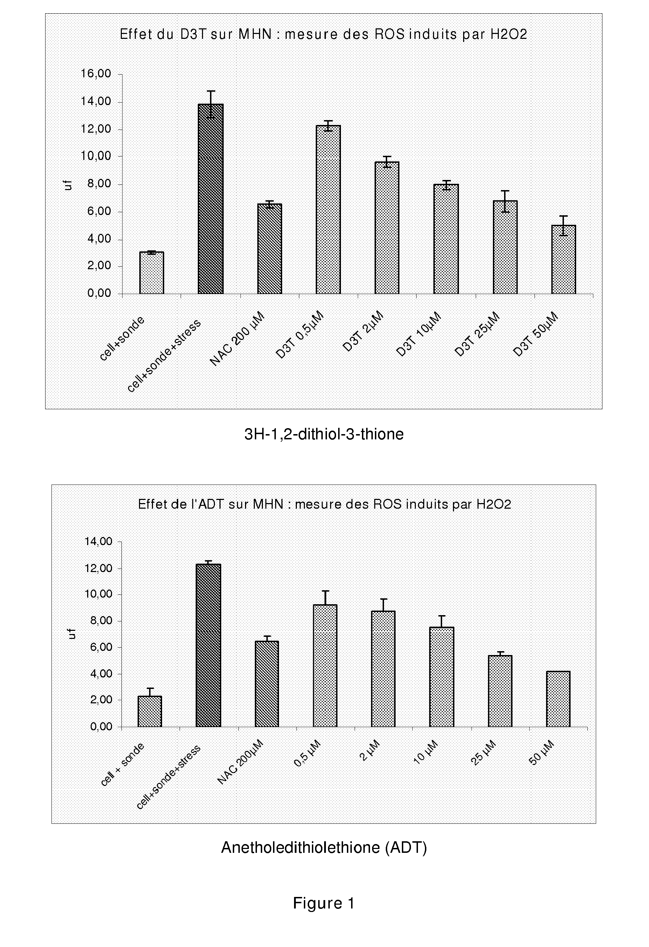

Les résultats représentent les données brutes de fluorescence auxquelles ont été retranchées les valeurs d'autofluorescence des cellules (« blanc »), exprimées en unité de fluorescence (uf), obtenues au cours d'une expérience représentative, voir la Figure 1. On observe bien une diminution des espèces réactives de l'oxygène en présente de D3T et d'ADT.

### 1-B-1. Protocole de mesure de la viabilité (contrôle de toxicité)

Les mélanocytes humains normaux (MHN) sont ensemencés à J0 à la densité de 4.10⁴ cellules/cm². Après adhésion des cellules (3h), le composé étudié est ajouté au milieu de culture. Après 24h à 48h, la viabilité cellulaire est mesurée à l'aide du bleu Alamar (UP669413 UPTIMA, Interchim) selon les instructions du fournisseur.

### 1-B-2. Résultats

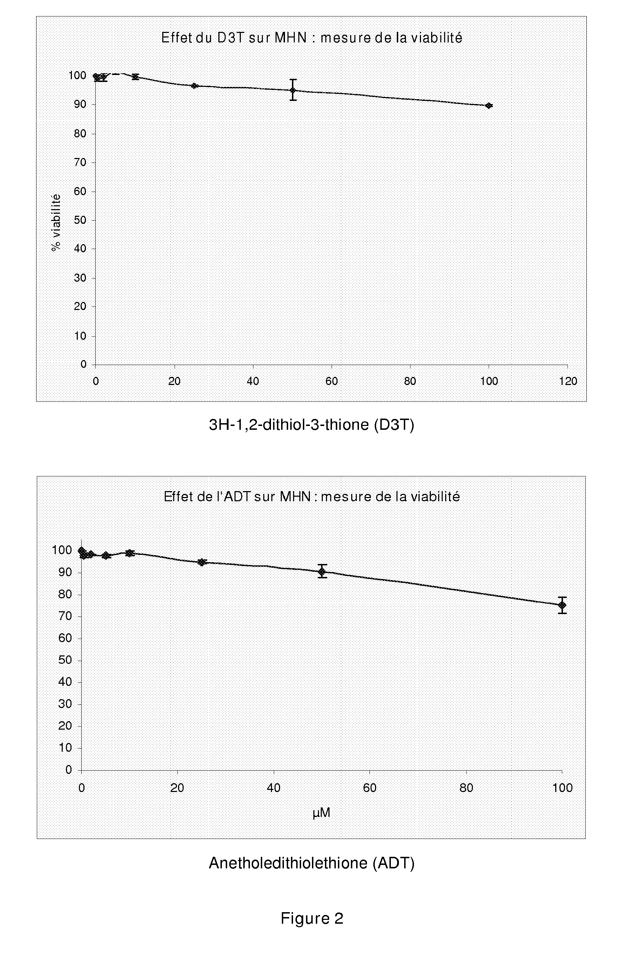

Les résultats représentent les pourcentages de signal fluorescent obtenus pour une expérience représentative (mesure en triplicate) (2 expériences indépendantes ont été réalisées) et présentés sous forme de courbe % de fluorescence en fonction de la concentration de l'actif étudié (en µM). Les résultats sont présentés en Figure 2.

### 1-C-1. Effet du 3H-1,2-dithiol-3-thione (D3T) sur la viabilité des mélanocytes humains normaux (MHN) en culture après un stress à la Dopamine

Les mélanocytes humains normaux (MHN) sont ensemencés à J0 à la densité de 10⁴ cellules/cm². Après adhésion des cellules (3h), l'actif étudié est ajouté au milieu de culture. Après 2 heures, la Dopamine est ajoutée au milieu de culture pour débuter le stress. A J3, la viabilité cellulaire est mesurée à l'aide du bleu Alamar (UP669413 UPTIMA, Interchim) selon les instructions du fournisseur.

### 1-C-2. Résultats

| Dopamine (µM) | Contrôle | NAC 200 µM | ADT 2 µM | ADT 15 µM |
|---|---|---|---|---|
| 0,00 | 100,00 | 100,00 | 100,00 | 100,00 |
| 20,00 | 98,48 | 101,99 | 97,48 | 92,68 |
| 40,00 | 96,04 | 101,66 | 100,00 | 95,22 |
| 60,00 | 88,41 | 99,67 | 98,74 | 95,54 |
| 70,00 | 74,09 | 95,68 | 95,90 | 94,90 |
| 80,00 | 71,34 | 89,37 | 92,43 | 94,90 |
| 90,00 | 60,06 | 77,74 | 82,02 | 87,58 |
| 100,00 | 23,32 | 23,82 | 51,42 | 81,21 |

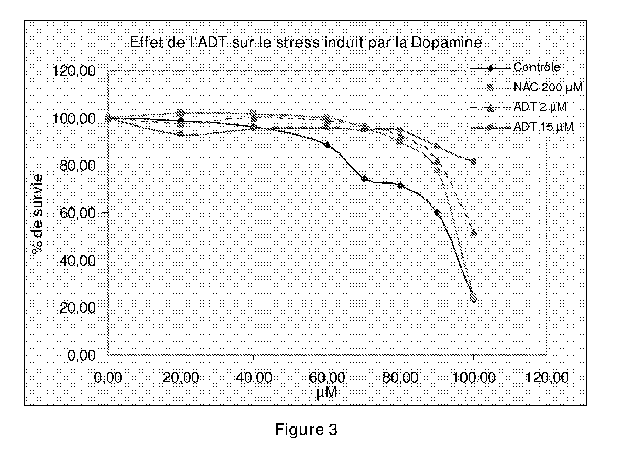

Les résultats sont représentés Figure 3. on observe que l'ADT augmente la survie des cellules avec une meilleure efficacité que la N-acétyl-cystéine qui est le composé de référence.

### Exemple 2 - Compositions

### - lotion capillaire

| | | |
|---|---|---|
| 3H-1,2-dithiole-3-thione | | 0,5 g |
| Propylène glycol | | 20 g |
| Ethanol 95° | | 30 g |
| Eau | qsp | 100 g |

Cette lotion est appliquée quotidiennement sur les zones à traiter et de préférence sur l'ensemble du cuir chevelu pendant au moins 10 jours et préférentiellement 1 à 2 mois. On constate alors une diminution de l'apparition des cheveux blancs ou gris et une repigmentation des cheveux gris.

### - shampooing traitant

| | | |
|---|---|---|
| sulforaphane | | 1,5 g |
| Polyglycéryl 3-hydroxylarylether | | 26 g |
| Hydroxy propyl cellulose vendue sous la dénomination de Klucell G par la société Hercules | | 2 g |
| Conservateurs | | qs |
| Ethanol 95° | | 50 g |
| Eau | qsp | 100 g |

Ce shampooing est utilisé à chaque lavage avec un temps de pose d'environ d'une minute.
Un usage prolongé, de l'ordre de deux mois, conduit au ralentissement de la canitie et à la repigmentation progressive des cheveux gris.
Ce shampooing peut également être utilisé à titre préventif afin de retarder le blanchissement des cheveux.

### - Gel traitant

| | | |
|---|---|---|
| phenetyl isothiocyanate | | 0,75 g |
| Huiles essentielles d'Eucalyptus | | 1 g |
| Econozole | | 0,2 g |
| Lauryl polyglyceryl 6 cetearyl glycoether | | 1,9 g |
| Conservateurs | | qs |
| Carbopol 934P vendu par la société BF Goodrich Corporation | | 0,3 g |
| Agent de neutralisation | | qs pH 7 |
| Eau | qsp | 100 g |

Ce gel est appliqué sur les zones à traiter deux fois par jour (matin et soir) avec un massage terminal. Après trois mois d'application, on observe une repigmentation des poils ou cheveux de la zone traitée.

### - capsules souples de gélatine

| | |
|---|---|
| Glucosinolate de sulforaphane | 0,05 g/capsule |
| Excipient | qsp |

On peut prendre une à trois capsules par jour.

## Revendications

1. Utilisation cosmétique d'au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés et leur mélange pour prévenir et/ou limiter et/ou arrêter le développement de la canitie.

2. Utilisation selon la revendication 1 pour maintenir et/ou restaurer la pigmentation naturelle des cheveux et/ou des poils gris.

3. Utilisation selon la revendication 1 ou 2, **caractérisée en ce que** ledit composé est administré par voie topique ou orale.

4. Composition cosmétique comprenant dans un milieu cosmétiquement acceptable au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés et leur mélange associé à un autre actif choisi parmi les agents de lutte des états desquamatifs du cuir chevelu et/ou des extraits végétaux à activité propigmentante.

5. Composition cosmétique comprenant dans un milieu cosmétiquement acceptable au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés et leur mélange associé à un agent ralentissant la chute des cheveux ou favorisant leur repousse.

6. Composition selon la revendication 4 ou 5, **caractérisée en ce que** ledit composé est encapsulé dans un enrobage tel que des microsphères, des nanosphères, des oléosomes ou des nanocapsules.

7. Composition selon l'une quelconque des revendications 4 à 6, **caractérisée en ce qu'**elle est adaptée à une application topique sur le cuir chevelu et/ou sur les zones de la peau recouvertes de poils.

8. Procédé de traitement cosmétique de la canitie, **caractérisé en ce qu'**on administre oralement ou qu'on applique topiquement sur la zone à traiter une composition comprenant au moins un composé choisi parmi le 3H-1,2-dithiole-3-thione, l'anethole dithiolethione, le sulforaphane, le phenetyl isothiocyanate, le 6-(methylsulphinyl)hexyl isothiocyanate, l'allyl isothiocyanate et leurs dérivés et leur mélange.
